# EUROPÄISCHE PATENTSCHRIFT

(11) **EP 1 056 708 B1**
(45) Veröffentlichungstag und Bekanntmachung des Hinweises auf die Patenterteilung: **21.11.2001**
(21) Anmeldenummer: 99907539.3
(22) Anmeldetag: 11.02.1999
(51) Int. Cl.: C07C 51/573, C07C 67/08, C07C 57/145, C07C 69/60, C07C 29/17, C07D 307/08, C07D 307/33, C07C 31/20

(54) **VERFAHREN ZUR ABTRENNUNG VON MALEINSÄUREANHYDRID AUS MALEINSÄUREANHYDRIDHALTIGEN GEMISCHEN DURCH STRIPPUNG**
METHOD FOR SEPARATING MALEIC ANHYDRIDE FROM MALEIC ANHYDRIDE-CONTAINING MIXTURES BY STRIPPING
PROCEDE DE SEPARATION DE L'ANHYDRIDE D'ACIDE MALEIQUE PAR DESORPTION A PARTIR DE MELANGES CONTENANT DE L'ANHYDRIDE D'ACIDE MALEIQUE

(30) Priorität: 13.02.1998 DE 19806038
(43) Veröffentlichungstag der Anmeldung: 06.12.2000
(73) Patentinhaber: BASF AKTIENGESELLSCHAFT, 67056 Ludwigshafen (DE)
(72) Erfinder: PINKOS, Rolf, D-67098 Bad Dürkheim (DE); RAHN, Ralf-Thomas, D-68167 Mannheim (DE)
(74) Vertreter: Isenbruck, Günter, Dr.
(86) Internationale Anmeldenummer: EP9900900
(87) Internationale Veröffentlichungsnummer: WO9941223

(56) Entgegenhaltungen:
- WO-A-97/43242
- DE-A- 3 521 768

## Beschreibung

Die Erfindung betrifft ein Verfahren zur Abtrennung von Maleinsäureanhydrid aus einem maleinsäureanhydridhaltigen Gasstrom, ein Verfahren zur Herstellung von Maleinsäureestern sowie ein Verfahren zur Herstellung von Hydrierungsprodukten von Maleinsäurederivaten, welche die Abtrennung von Maleinsäureanhydrid aus einem maleinsäureanhydridhaltigen Gasstrom umfassen.

Maleinsäureanhydrid wird großtechnisch durch katalytische Luftoxidation von Kohlenwasserstoffen, wie Benzol, Butenen oder Butan hergestellt. Das dabei erhaltene gasförmige Reaktionsgemisch enhält neben Maleinsäureanhydrid vor allem Wasser, Kohlenmonoxid und Kohlendioxid.

Üblicherweise wird Maleinsäureanhydrid aus den maleinsäureanhydridhaltigen Reaktorabgasen der Kohlenwasserstoff-Oxidation durch Quenchen mit hochsiedenden aliphatischen Alkoholen herausgewaschen. Ein solches Verfahren ist beispielsweise in DE-A-31 06 819 beschrieben. Nachteilig an diesem Verfahren ist, daß bei dem Quenchprozeß ein Teil des Alkohols mit dem Abgas verlorengeht. Diese Verluste verursachen hohe Kosten

Das aus den Reaktorabgasen der Kohlenwasserstoff-Oxidation erhältliche Maleinsäureanhydrid, bzw. die aus Maleinsäureanhydrid durch Umsetzung mit flüssigen Absorptionsmitteln erhaltenen Maleinsäurederivate, wie Maleinsäureester, werden häufig einer nachfolgenden Hydrierung zu Butandiol, Tetrahydrofuran oder γ-Butyrolacton unterworfen. In WO 97/43234 ist ein Verfahren zur Herstellung von γ-Butyrolacton, Butan-1,4-diol und Tetrahydrofuran beschrieben, bei dem Maleinsäureanhydrid aus den maleinsäureanhydridhaltigen Reaktorabgasen der Kohlenwasserstoff-Oxidation mit einem hochsiedenden inerten organischen Lösungsmittel als Absorptionsmittel ausgewaschen und Maleinsäureanhydrid aus dem erhaltenen Absorptionsaustrag durch Strippung mit einem Wasserstoffgasstrom abgetrennt wird. Das herausgestrippte Maleinsäureanhydrid gelangt nachfolgend in eine Gasphasenhydrierung zur Herstellung von Butandiol, Tetrahydrofuran und Y-Butyrolacton. Nachteilig an diesem Verfahren ist die Bildung von freier Maleinsäure bzw. Fumarsäure. In Gegenwart selbst kleiner Mengen Wasser, wie sie in den Reaktorabgasen der Kohlenwasserstoff-Oxidation häufig enthalten sind, wird aus Maleinsäureanhhydrid die freie Maleinsäure gebildet, die korrosiv ist. Die freie Maleinsäure neigt ferner zur Isomerisierung in die nur sehr schlecht lösliche Fumarsäure, was im nachfolgenden Hydrierungsschritt zu erheblichen Problemen durch Ablagerungen auf dem Hydrierkatalysator führen kann. Femer wird im Hydrierungsschritt aus Malein- bzw. Fumarsäure die Bemsteinsäure gebildet, die extrem schwerflüchtig ist, was früher oder später zur Verbackung des Katalysators führen kann.

Aufgabe der Erfindung ist es, ein für die Herstellung von Maleinsäurederivaten bzw. von deren Hydrierungsprodukten geeignetes Verfahren zur Abtrennung von Maleinsäureanhydrid aus den maleinsäureanhydridhaltigen Reaktorabgasen der Kohlenwasserstoff-Oxidation bereitzustellen, welches die vorstehend genannten Nachteile vermeidet.

Die Erfindung geht aus von einem Verfahren zur Abtrennung von Maleinsäureanhydrid aus einem maleinsäureanhydridhaltigen Gasstrom, bei dem der maleinsäureanhydridhaltige Gasstrom mit einer flüssigen Absorbensphase, enthaltend mindestens ein hochsiedendes inertes Absorptionsmittel für Maleinsäureanhydrid, in Berührung gebracht wird, und aus der erhaltenen flüssigen Absorbatphase Maleinsäureanhydrid durch Dampf-Strippen abgetrennt wird. Das erfindungsgemäße Verfahren ist dadurch gekennzeichnet. daß als Strippungsmittel ein Alkohol eingesetzt wird, wobei Maleinsaureanhydrid zumindest teilweise mit dem Alkohol reagiert.

Unter Absorbensphase wird die flüssige Mischung aus Absorptionsmitteln verstanden. Die Absorbatphase ist die mit dem Absorptiv, hier Maleinsäureanhydrid bzw. dessen Umsetzungsprodukte, beladene Absorbensphase.

In Gegenwart von Alkohol als Strippungsmittel findet zumindest teilweise eine Alkoholyse des Maleinsäureanhydrids unter Bildung des Monoesters und darüber hinaus eine weitergehende Versterung unter Bildung des Diesters statt. Dadurch wird die Hydrolyse des Anhydrids zur freien Maleinsäure durch aus dem Gasstrom absorbierte Wasserspuren zugunsten der Esterbildung zurückgedrängt. Eventuell durch die Hydrolyse gebildete Maleinsäure reagiert nachfolgend zum Maleinsäuremono- bzw. -diester. Dadurch ist der Anteil an freier Säure in der Absorbatphase gering.

Die flüssige Absorbens- bzw. Absorbatphase enthält mindestens ein hochsiedendes inertes Absorptionsmittel für Maleinsäureanhydrid. Das inerte hochsiedende Absorptionsmittel weist im allgemeinen einen Siedepunkt auf, der um mindestens 30°C, bevorzugt um mindestens 50°C, besonders bevorzugt um mindestens 70°C höher liegt als der Siedepunkt von Maleinsäureanhydrid. Geeignete Absorptionsmittel sind beispielsweise in WO 97/43234 beschrieben. Beispiele sind hochsiedende Ester der Phthalsäure, Terephthalsäure oder Maleinsäure, wie Dimethyl-, Diethyl- oder Dibutylphthalate, Dimethylterephthalat oder Dibutylmaleat, aromatische Kohlenwasserstoffe, wie Dibenzylbenzol, Ester von cycloaliphatischen Säuren wie Dibutylhexahydrophthalat, ferner Polymethylbenzophenone. Ethylenglycolether und Polysiloxanether. Bevorzugte Absorptionsmittel sind Ester von aromatischen bzw, cycloaliphatischen Dicarbonsäuren, besonders bevorzugt sind Ester der Phthalsäure bzw. Terephthalsäure. Hochsiedendes Absorptionsmittel liegt in der Absorbatphase im allgemeinen im Überschuß vor. Das Gewichtsverhältnis Absorptionsmittel zu Maleinsäureanhydrid beträgt im allgemeinen von 1 : 1 bis 100 : 1, bevorzugt von 2 : 1 bis 100 : 1 , besonders bevorzugt von 4 : 1 bis 100 : 1.

Die Abtrennung von Maleinsäureanhydrid aus der Absorbatphase erfolgt durch Dampf-Strippung mit einem Alkohol als Strippungsmittel. Unter Strippung wird die Abtrennung des in der Absorbatphase vorliegenden Absorptivs mit Hilfe eines Desorptionshilfsmittels (Strippungsmittel) verstanden, wobei sich das Absorptiv in dem Strippungsmittel anreichert. Die Absorbatphase wird an dem Absorptiv entsprechend abgereichert und wird dabei regeneriert. In dem vorliegenden Verfahren findet zusätzlich zu dem Stoffübergang aus der Absorbatphase zumindest teilweise eine Reaktion des Absorptivs mit dem Strippungsmittel statt.

Als Strippungsmittel sind für das erfindungsgemäße Verfahren prinzipiell alle unter den Verfahrensbedingungen flüchtigen Alkohole geeignet (Strippungsalkohole). Bevorzugt werden Methanol, Ethanol, n-Propanol, iso-Propanol, n-Butanol, iso-Butanol, sek.-Butanol, n-Pentanol und iso-Pentanol, besonders bevorzugt Methanol, Ethanol und n-Butanol eingesetzt. Das Molverhältnis Alkohol zu Maleinsäureanhydrid beträgt im allgemeinen von 1 : 1 bis
300 : 1, bevorzugt 1,5 : 1 bis 200 : 1, besonders bevorzugt 3 : 1 bis
50 : 1.

Die Strippung wird im allgemeinen im Gegenstromverfahren durchgeführt. Dabei läuft die mit Maleinsäureanhydrid beladene Absorbatphase dem dampfförmigen Strippungsmittel entgegen, wobei ein intensiver Stoff- und Wärmeaustausch zwischen der absteigenden flüssigen Absorbatphase und der aufsteigenden Dampfphase stattfindet. Die flüssige Absorbatphase wird sukzessive an Maleinsäureanhydrid abgereichert, die Dampfphase entsprechend an Maleinsäureanhydrid bzw, deren Reaktionsprodukten angereichert. Vorzugsweise wird die Strippung im Gegenstromverfahren in einer Kolonne, beispielsweise einer Füllkörperkolonne oder Glockenbodenkolonne, durchgeführt. Vorzugsweise werden der als Strippungsmittel eingesetzte Alkohol im unteren Abschnitt der Kolonne und die mit Maleinsäureanhydrid beladene Absorbatphase im oberen Abschnitt der Kolonne in die Kolonne eingebracht. Am Sumpf der Kolonne wird die an Maleinsäureanhydrid abgereicherte Absorbatphase, am Kopf der Kolonne der als Strippungsmittel eingesetzte Alkohol zusammen mit den Maleinsäurederivaten erhalten.

Unter Maleinsäurederivaten im Sinne der Erfindung werden Maleinsäureanhydrid, Maleinsäure, Maleinsäuremonoester, Maleinsäurediester, Fumarsäure, Fumarsäuremonoester und Fumarsäurediester, wobei den Estern die zur Strippung eingesetzten Alkohole zugrundeliegen, verstanden.

Es ist auch möglich, die Abtrennung von Maleinsäureanhydrid aus der Absorbatphase in einem Verweilzeitbehälter mit aufgesetztem Destillationskopf durchzuführen, wobei der Alkohol zusammen mit oder getrennt von der Absorbatphase in den Behälter geführt werden kann. Die Maleinsäurederivate gehen mit dem verdampfenden Alkohol über den Destillationskopf über.

Das Arbeiten im Gegenstromverfahren entspricht einer bevorzugten Ausführungsform der Erfindung. In einer besonders bevorzugten Ausführungsform befinden sich zwischen der Zulaufstelle für die Absorbatphase und dem Kolonnenkopf weitere Trennstufen zur Zurückhaltung des hochsiedenden inerten Absorptionsmittels. Der Absorbatphase kann vor der Einspeisung in die Kolonne ein Teil des Strippungsalkohols zugesetzt werden. Im Sumpf wird die abgereicherte Absorbatphase erhalten, die einer erneuten Absorption zugeführt werden kann. Ein hoher Abreicherungsgrad ist nicht erforderlich, aber wünschenswert. Er beträgt, bezogen auf den Maleinsäureanhydrid-Gehalt des Kopfzulaufes, im allgemeinen 80 bis 100, bevorzugt 95 bis 100%.

Die Strippung wird im allgemeinen bei Temperaturen oberhalb 100°C durchgeführt. Vorzugsweise beträgt sie bei der Strippung 150 bis 300°C, besonders bevorzugt 170 bis 300°C. Der Druck beträgt bei der Strippung im allgemeinen zwischen 10 mbar und 10 bar, bevorzugt zwischen 100 mbar und 6 bar, besonders bevorzugt zwischen 300 mbar und 5 bar.

Während der Strippung wird Maleinsäureanhydrid zumindest teilweise mit dem Alkohol verestert. Dabei wird ein Gemisch gebildet, welches im wesentlichen aus Maleinsäureanhydrid, Maleinsäure, Fumarsäure, Maleinsäuremonoester, Maleinsäurediester, Fumarsäuremonoester und Fumarsäurediester besteht. Im allgemeinen gilt, daß ein hoher Überschuß an Strippungsalkohol die Esterbildung begünstigt. Vorteilhaft an dem erfindungsgemäßen Verfahren ist der geringe Anteil an freier Malein- bzw. Fumarsäure im Kopfprodukt. Im allgemeinen ist der Anteil an freier Säure im Kopfprodukt, bezogen auf die Gesamtmenge der Maleinsäurederivate, abhängig von der vorhandenen Wassermenge.

Ferner ist vorteilhaft, daß der Anteil an Fumarsäurestern im Kopfprodukt gering ist. Der Anteil an Fumarsäuremono- bzw. -diester beträgt, bezogen auf die Gesamtmenge an Maleinsäurederivaten, 0 bis 10 Gew.-%, bevorzugt 0 bis 5 Gew.-%.

Gegenstand der Erfindung ist weiterhin ein Verfahren zur Herstellung von Maleinsäureestern mit den Schritten:
a) Inberührungbringen eines maleinsäureanhydridhaltigen Gastroms mit einer flüssigen Absorbensphase, enthaltend mindestens ein inertes hochsiedendes Absorptionsmittel mit einem um mindestens 30°C höheren Siedepunkt als Maleinsäureanhydrid,
b) Abtrennen von Maleinsäureanhvdrid durch Strippung mit Alkohol als Strippungsmittel, wobei Maleinsäureanhydrid zumindest teilweise mit dem Alkohol reagiert,
c) gegebenenfalls weitergehende Veresterung mit dem Alkohol.

Vorzugsweise wird in Schritt a) ein Gasstrom aus maleinsäureanhydridhaltigen Reaktorabgasen aus der Maleinsäureanhydrid-Herstellung durch Kohlenwasserstoff-Oxidation eingesetzt.

Im Abtrennschritt b) wird im allgemeinen ein Gemisch erhalten, welches, bezogen auf die Gesamtmenge an Maleinsäureester, 5 bis 95 Gew.-%, bevorzugt 10 bis 90 Gew.-% des Maleinsäurediesters enthält. Vorzugsweise schließt sich an den Abtrennschritt b) ein Schritt c) an, in dem der Anteil an Maleinsäuremonoester und ggf. Maleinsäureanhydrid bzw. Maleinsäure bzw. Fumarsäure und Fumarsäuremonoester durch weitergehende Veresterung zum Maleinsäurediester bzw. Fumarsäurediester verringert wird. Dabei wird vorzugsweise mit dem Strippungalkohol verestert. Die Veresterung von Maleinsäureanhydrid, Maleinsäure, Maleinsäuremonoester bzw. allgemein von Carbonsäuren ist in EP-A 0 255 399, EP-A 0 454 719, DE-A 5 543 673, DE-A 19 607 953 oder in "Organikum", VEB Deutscher Verlag der Wissenschaften, 15. Auflage, Berlin 1977, S. 498-502 beschrieben. Bevorzugt werden mit dem erfindungsgemäßen Verfahren die Maleinsäurediester hergestellt.

Das im Abtrennschritt b) erhaltene Gemisch bzw. das durch weitergehende Veresterung mit dem Strippungsalkohol gemäß Schritt c) des vorstehend beschriebenen Verfahrens erhaltene Gemisch bzw. aus diesem Gemisch durch Destillation gewonnene Maleinsäurederivate können nachfolgend einer Hydrierung unterworfen werden. Je nach Reaktionsbedingungen werden bevorzugt 1,4-Butandiol, Tetrahydrofuran und/oder γ-Butyrolacton erhalten.

Gegenstand der vorliegenden Erfindung ist auch ein Verfahren zur Herstellung von mindestens einer Verbindung ausgewählt aus der Gruppe bestehend aus 1,4-Butandiol, γ-Butyrolacton und Tetrahydrofuran mit den Schritten:
a) Inberührungbringen eines maleinsäureanhydridhaltigen Gastroms mit einer flüssigen Absorbensphase, enthaltend mindestens ein mertes hochsiedendes Absorptionsmittel mit einem um mindestens 30°C höheren Siedepunkt als Maleinsäureanhydrid.
b) Abtrennen von Maleinsäureanhydrid durch Strippung mit Alkohol als Strippungsmittel, wobei Maleinsäureanhydrid zumindest teilweise mit dem Alkohol reagiert,
c) gegebenenfalls weitergehende Veresterung mit dem Alkohol.
d) gegebenenfalls Abtrennung des Alkohols von dem erhaltenen Gemisch, enthaltend die Derivate der Maleinsäure,
e) Hydrierung der erhaltenen Maleinsäurederivate.

Vorzugsweise wird in Schritt a) ein Gasstrom aus maleinsäureanhydridhaltigen Reaktorabgasen aus der Maleinsäureanhydrid-Herstellung durch Kohlenwasserstoff-Oxidation eingesetzt.

Die im Abtrennschritt b) erhaltenen Maleinsäurederivate können direkt hydriert werden. Diese können im Gemisch mit dem Strippungsalkohol vorliegen. Vorteilhaft, für das Gelingen der Hydrierung jedoch nicht entscheidend, ist ein hoher Veresterungsgrad der erhaltenen Maleinsäurederivate. Im allgemeinen liegen die zu hydrierenden Maleinsäurederivate zu mindestens 50 Gew.-%, bevorzugt zu mindestens 70 Gew.-%. besonders bevorzugt zu mindestens 90 Gew.-%, speziell bevorzugt zu mindestens 95 Gew.-%, insbesondere zu mindestens 97 Gew.-% in Form der Maleinsäurediester vor. Zur Erzielung eines hohen Veresterungsgrades wird vorzugsweise das im Abtrennschritt b) erhaltene Gemisch einer weitergehenden Veresterung mit dem Alkohol unterzogen.

Das im Abtrennschritt b) bzw. durch weitergehende Veresterung gemäß Schritt c) erhaltene Gemisch kann direkt oder nach Abtrennung des als Strippungsmittel eingesetzten Alkohols der Hydrierung unterworfen werden. Vorzugsweise wird vor der Hydrierung der Alkohol gemäß Schritt d) aus dem erhaltenen Gemisch abgetrennt.

Die Hydrierung der erhaltenen Maleinsäurederivate kann in flüssiger Phase oder in der Gasphase durchgeführt werden. Die Hydrierung kann mit homogen gelösten Katalysatoren, mit suspendierten Katalysatoren oder mit Festbettkatalysatoren durchgeführt werden. Im allgemeinen enthalten die eingesetzten Hydrierkatalysatoren eines oder mehrere der folgenden Elemente:
Kupfer, Palladium, Platin, Ruthenium, Rhenium, Kobalt, Mangan, Nickel, Molybdän und Chrom. Solche Hydrierkatalysatoren und die mit ihnen durchgeführten Hydrierungen sind beispielsweise in WO 97/43234,
EP-A 0 552 463, EP-A 0 724 908, DE-A 2 501 499, BE 851 227,
US 4,115, 919, EP-A 0 147 219, EP-A 0 417 867, US 5,115,086 und
EP-A 0 382 050 beschrieben. Bei der Gasphasenhydrierung beträgt die Temperatur im allgemeinen 150 bis 250°C und der Druck 5 bis 100 bar. Bei Hydrierung in flüssiger Phase beträgt die Temperatur vorzugsweise 100 bis 300°C und der Druck 60 bis 300 bar.

Der in den Maleinsäureestem gebundene Alkohol wird bei der Hydrierung freigesetzt und kann wiedergewonnen und erneut zur Strippung eingesetzt werden.

Das erfindungsgemäße Verfahren zeichnet sich dadurch aus, daß die Bildung korrosiver Säuren weitgehend unterdrückt wird. Femer sind die gebildeten Maleinsäure- bzw. Fumarsäureester bzw. aus diesen durch Hydrierung gebildete Bemsteinsäureester gegenüber den freien Säuren vergleichsweise gut löslich und leichtflüchtig, so daß Probleme durch Ablagerungen auf dem Hydrierkatalysator vermieden werden.

Die Erfindung wird durch die nachstehenden Beispiele näher erläutert.

### Beispiele

Die nachfolgend beschriebenen Versuche werden in einer Apparatur durchgeführt, die aus einem beheizbaren Destillationskolben von 250 ml Fassungsvermögen, mit Sumpfzulauf und Sumpfablauf, einer aufgesetzten, beheizbaren Füllkörperkolonne von 50 cm Länge und 3 cm Weite und einem Destillationsaufsatz mit Kopfabzug besteht. Zwischen dem oberen Ende der beheizbaren Kolonne und dem Destillationsaufsatz befindet sich der Zulauf des Absorbats, einer Mischung aus Dimethylphthalat als Absorptionsmittel und Maleinsäureanhydrid. Über den Sumpf wird Methanol als Strippungsalkohol eingeleitet. Die Zu- und Abläufe erfolgen kontinuierlich, der Sumpfstand wird absatzweise abgelassen. Nach einer Betriebszeit von 5 Stunden werden Proben des Sumpfaustrages und des Kopfaustrages genommen und gaschromatographisch analysiert. Die %-Angaben beziehen sich auf GC-Flächen-%.

### Beispiel 1

In den Sumpf werden 150 g Dimethylphthalat vorgelegt und auf 220°C aufgeheizt. Danach werden 100 ml Methanol/h kontinuierlich in den Sumpf eingeleitet und verdampft. Die Füllkörperkolonne wird auf 220°C aufgeheizt, wobei sich im Kopf der Kolonne eine Temperatur von ca. 65°C einstellt. Danach werden 100 ml/h eines Gemsiches aus 80 Gew.-% Dimethylphthalat und 20 Gew.-% Maleinsäureanhydrid als Kopfzulauf kontinuierlich zugeführt, wobei die Temperatur im Kolonnenkopf auf 140 bis 150°C steigt. Der Sumpfaustrag enthält 98 bis 99% Dimethylphthalat, ca. 0,3% Methanol, ca. 0.3% Maleinsäureanhydrid, ca. 0,28% Maleinsäuremono- und -dimethylester. Der Kopfaustrag enthält ca. 0,8% Maleinsäureanhydrid, ca. 20% Maleinsäuremonomethylester, ca. 14% Maleinsäuredimethylester sowie ca. 62% Methanol. Freie Maleinsäure bzw. Fumarsäure konnte nicht nachgewiesen werden.

### Beispiel 2

Es wird wie in Beispiel 1 beschrieben verfahren, der Kopfzulauf besteht jedoch aus 100 ml/h eines Gemsiches aus 78.5 Gew.-% Dimethylphthalat, 19,5 Gew.-% Maleinsäureanhydrid und 2 Gew.-% Wasser, der Sumpfzulauf besteht aus 200 ml/h Methanol. Die Temperatur am Kolonnenkopf beträgt ca. 150°C. Der Sumpfaustrag enthält 99% Dimethylphthalat, ca. 0,5% Methanol, < 0.05% Maleinsäuremono und Dimethylester sowie in Spuren Maleinsaureanhydrid (< 0.05%). Der Kopfaustrag enthält < 0,05% Maleinsäureanhydrid, ca. 18% Maleinsäuremonomethylester, ca. 1% Maleinsäuredimethylester, ca. 77% Methanol. Der Rest besteht aus Fumarsäuremonomethylester (ca. 1%) sowie Maleinsäure (0,4%) und Fumarsäure (< 0,05%).

### Beispiel 3

Es wird wie in Beispiel 1 verfahren, jedoch besteht der Kopfzulauf aus 100 ml/h einer Mischung aus 79,7 Gew.-% Dimethylphthalat, 19,9 Gew.-% Maleinsäureanhydrid und 0,4 Gew.-% Wasser, der Sumpfzulauf besteht aus 100 ml/h Methanol. Am Kolonnenkopf stellt sich eine Temperatur von ca. 150°C ein. Der Sumpfaustrag enthält ca. 99,2% Dimethylphthalat, ca. 0,3% Methanol, < 0,05% Maleinsäuremono bzw. Dimethylester und < 0,05% Maleinsäureanhydrid. Der Kopfaustrag enthält < 0,05% Maleinsäureanhydrid, ca. 14% Maleinsäuremonomethylester, ca. 11% Maleinsäuredimethylester, ca. 2% Fumarsäuremonomethylester, ca. 72% Methanol. Freie Maleinsäure (ca. 0,5%) bzw. Fumarsäure (< 0,05%) lagen nur in kleinen Mengen vor.

## Patentansprüche

1. Verfahren zur Abtrennung von Maleinsäureanhydrid aus einem maleinsäureanhydridhaltigen Gasstrom, bei dem
a) der maleinsäureanhydridhaltige Gasstrom mit einer flüssigen Absorbensphase, enthaltend mindestens ein hochsiedendes inertes Absorptionsmittel für Maleinsäureanhydrid, in Berührung gebracht wird und
b) aus der erhaltenen flüssigen Absorbatphase Maleinsäureanhydrid durch Dampf-Strippung abgetrennt wird,
**dadurch gekennzeichnet, daß** als Strippungsmittel ein Alkohol eingesetzt wird, wobei Maleinsäureanhydrid zumindest teilweise mit dem Alkohol reagiert.

2. Verfahren nach Anspruch 1, **dadurch gekennzeichnet, daß** der als Strippungsmittel eingesetzte Alkohol ausgewählt ist aus der Gruppe bestehend aus Methanol, Ethanol und n-Butanol.

3. Verfahren nach Anspruch 1 oder 2, **dadurch gekennzeichnet, daß** als Gasstrom maleinsäureanhydridhaltige Reaktorabgase aus der Maleinsäureanhydrid-Herstellung durch Kohlenwasserstoff-Oxidation eingesetzt werden.

4. Verfahren nach einem der Ansprüche 1-3, **dadurch gekennzeichnet, daß** das hochsiedende inerte Absorptionsmittel einen um mindestens 30°C höheren Siedepunkt als Maleinsäureanhydrid aufweist.

5. Verfahren nach Anspruch 4 zur Herstellung von Estern der Maleinsäure, gegebenenfalls mit dem zusätzlichen Schritt:
c) weitergehende Veresterung des im Abtrennschritt b) erhaltenen Gemischs mit dem Alkohol.

6. Verfahren nach Anspruch 5 zur Herstellung von Diestern der Maleinsäure.

7. Verfahren nach Anspruch 4 zur Herstellung von mindestens einer Verbindung ausgewählt aus der Gruppe bestehend aus 1,4-Butandiol, γ-Butyrolacton und Tetrahydrofuran mit den zusätzlichen Schritten:
c) gegebenenfalls weitergehende Veresterung mit dem Alkohol.
d) gegebenenfalls Abtrennung des Alkohols von den erhaltenen Gemisch. enthaltend Derivate der Maleinsäure,
e) Hydrierung der erhaltenen Maleinsäurederivate.

## Claims

1. A process for the separation of maleic anhydride from a maleic anhydride-containing gas stream in which
a) the maleic anhydride-containing gas stream is brought into contact with a liquid absorbent phase containing at least one high-boiling, inert absorbent for maleic anhydride, and
b) maleic anhydride is separated from the resultant liquid absorbate phase by vapor stripping,
wherein the stripping agent used is an alcohol, at least some of the maleic anhydride reacting with the alcohol.

2. A process as claimed in claim 1, wherein the alcohol employed as a stripping agent is selected from the group consisting of methanol, ethanol and n-butanol.

3. A process as claimed in claim 1 or 2, wherein the gas stream employed is a maleic anhydride-containing reactor offgas from the preparation of maleic anhydride by the oxidation of hydrocarbons.

4. A process as claimed in any one of claims 1 to 3, wherein the high-boiling, inert absorbent has a boiling point which is at least 30°C higher than that of maleic anhydride.

5. A process as claimed in claim 4 for the preparation of esters of maleic acid, if desired comprising the additional step:
c) further esterification of the mixture obtained in the separation step b), using the alcohol.

6. A process as claimed in claim 5 for the preparation of diesters of maleic acid.

7. A process as claimed in claim 4 for the preparation of at least one compound selected from the group consisting of 1,4-butanediol, γ-butyrolactone and tetrahydrofuran, comprising the additional steps:
c) if desired, further esterification using the alcohol,
d) if desired, separation of the alcohol from the resultant mixture containing maleic acid derivatives,
e) hydrogenation of the resultant maleic acid derivatives.

## Revendications

1. Procédé de séparation de l'anhydride maléique à partir d'un courant de gaz contenant de l'anhydride maléique, comprenant les étapes consistant à :
a) mettre en contact un courant de gaz contenant de l'anhydride maléique avec un produit absorbant en phase liquide contenant au moins un agent d'absorption pour l'anhydride maléique, à haut point d'ébullition et inerte, et
b) séparer l'anhydride maléique à partir du produit absorbé obtenu en phase liquide à l'aide d'un entraînement à la vapeur,
**caractérisé en ce que** l'on met en oeuvre, en tant qu'agent d'entraînement, un alcool, et **en ce que** l'anhydride maléique réagit au moins partiellement avec l'alcool.

2. Procédé selon la revendication 1, **caractérisé en ce que** l'alcool mis en oeuvre en tant qu'agent d'entraînement est choisi dans le groupe formé par le méthanol, l'éthanol et le n-butanol.

3. Procédé selon la revendication 1 ou 2, **caractérisé en ce que** l'on met en oeuvre en tant que courant de gaz, des effluents gazeux de réacteur contenant de l'anhydride maléique et provenant de la fabrication de l'anhydride maléique au moyen de l'oxydation d'hydrocarbures.

4. Procédé selon une quelconque des revendications 1 à 3, **caractérisé en ce que** l'agent d'absorption à haut point d'ébullition et inerte présente un point d'ébullition supérieur d'au moins 30 °C à celui de l'anhydride maléique.

5. Procédé de fabrication d'esters de l'acide maléique selon la revendication 4, comprenant éventuellement l'étape supplémentaire suivante consistant à :
estérifier ultérieurement le mélange obtenu à l'étape de séparation b), avec l'alcool.

6. Procédé selon la revendication 5, pour la fabrication de diesters de l'acide maléique.

7. Procédé selon la revendication 4, pour la fabrication d'au moins un composé choisi dans le groupe formé par le 1,4-butanediol, la γ-butyrolactone, et le tétrahydrofuranne, comprenant les étapes supplémentaires suivantes consistant à :
a) éventuellement estérifier ultérieurement avec l'alcool,
b) éventuellement' séparer l'alcool du mélange obtenu contenant les dérivés de l'acide maléique,
c) hydrogéner les dérivés de l'acide maléique obtenus.
